# EUROPEAN PATENT SPECIFICATION

(11) **EP 1 951 268 B1**
(45) Date of publication and mention of the grant of the patent: **07.06.2017**
(21) Application number: 06799011.9
(22) Date of filing: 29.09.2006
(51) Int. Cl.: A61K 35/12

(54) **USE OF MESENCHYMAL STEM CELLS GENETICALLY MODIFIED TO EXPRESS A SUICIDE GENE FOR TREATING A CANCER**
VERWENDUNG VON GENETISCH MODIFIZIERTEN MESENCHYMALEN STAMMZELLEN ZUR EXPRESSION EINES SUIZIDGENS ZUR BEHANDLUNG VON KREBS
UTILISATION DE CELLULES SOUCHES MÉSENCHYMATEUSES GÉNÉTIQUEMENT MODIFIÉES POUR EXPRIMER UN GÈNE SUICIDE PERMETTANT DE TRAITER UN CANCER

(30) Priority: 29.09.2005 KR 20050091155
(43) Date of publication of application: 06.08.2008
(73) Proprietor: Ajou University Industry-Academic Cooperation Foundation, Gyeonggi-do 16499 (KR)
(72) Inventor: SUH, Hae-Young, Suwon-si Gyeonggi-do 443 -738 (KR); CHANG, Da-Young, Gyeonggi-do 448-130 (KR); KIM, Sung-Soo, Seoul 134-845 (KR); YOO, Seung-Wan, Seoul 140-030 (KR); LEE, Young-Don, Suwon-si Gyeonggi-do 442-762 (KR)
(74) Representative: Grünecker Patent- und Rechtsanwälte PartG mbB
(86) International application number: PCT/KR2006/003928
(87) International publication number: WO 2007/037653

(56) References cited:
- WO-A-99/30648
- WO-A-99/37332
- WO-A2-99/43286
- US-A- 5 486 359
- US-B1- 6 464 983
- US-B2- 6 905 678
- OLIVIER JUFFROY ET AL: "Subcutaneous graft of D1 mouse mesenchymal stem cells leads to the formation of a bone-like structure", DIFFERENTIATION, vol. 78, no. 4, 1 November 2009 (2009-11-01), pages 223-231, XP055075939, ISSN: 0301-4681, DOI: 10.1016/j.diff.2009.07.005
- YOSHITOMO HONDA ET AL: "Guiding the osteogenic fate of mouse and human mesenchymal stem cells through feedback system control", SCIENTIFIC REPORTS, vol. 3, 5 December 2013 (2013-12-05), XP055220584, DOI: 10.1038/srep03420

## Description

### FIELD OF THE INVENTION

The present invention relates to the use of a mesenchymal stem cell expressing a suicide gene for the manufacture of a medicament for treating a cancer, wherein the suicide gene is a gene encoding cytosine deaminase, and wherein cytosine deaminase expressed from the suicide gene converts the 5-fluorocytosine to 5-fluorouracil, wherein the mesenchymal stem cell is a human mesenchymal stem cell isolated from bone marrow, peripheral blood or caught blood, and wherein the cancer is selected from brain cancer, breast cancer, liver cancer, pancrease cancer, colorectal cancer and lung cancer, and a corresponding mesenchymal stem cell expressing a suicide gene for use in treating a cancer.

### BACKGROUND OF THE INVENTION

Glioblastoma is one of the most difficult cancers due to its metastasis to other sites and no effective treatments are currently available. Conventional excision of the brain tumor cannot be performed safely in most cases and the chemotherapy with anti-cancer medicines is not effective due to the brain-blood barrier (BBB) which blocks penetration of drugs into the brain parenchyma.

Also, gene therapy for the treatment of glioblastoma by introducing viruses engineered to carry an anti-cancer gene directly into the tumor cells is not effective when a number of tiny tumors are involved. Further, repeated injections of virus may cause severe immune rejections.

It has recently been reported that the neural stem cells (Aboody et al., Proc. Natl. Acad. Sci. USA, 97, 12846-12851 (2000); Brown et al., Human Gene Therapy, 14, 1777-1785 (2003); Tang et al., Human Gene Therapy, 14, 1247-1254, (2003) and Zhang et al., NeuroImage, 23, 281-287 (2004)) and mesenchymal stem cells (Nakamura et al., Gene Therapy, 11, 1155-1164 (2004) and Zhang et al., NeuroImage, 23, 281-287 (2004)) exhibited tropism toward glioblastoma, and therefore, there have been attempts to utilize such stem cells as gene carriers.

For example, it has been reported that the use of neural stem cells which express cytosine deaminase (CD), a suicidal gene of *E. coli,* showed excellent anti-cancer effects in the treatment of glioblastoma (Aboody *et al*., *supra* and Brown *et al*., *supra*)*.* However, it is very difficult to obtain neural stem cells because it must be isolated and cultured from the brain tissue of aborted fetus. Thus, cell lines immortalized by an oncogene were used in the above experiments, which could induce a cancer and immune rejection.

It has been reported that the injection of mesenchymal stem cells (MSCs) expressing interleukin-2 (IL-2) into rat glioma model augments the antitumor effect and prolonged the survival of tumor-bearing rats (Nakamura *et al*., *supra*)*.* However, above method has the problems that it is difficult for the immune cells to penetrate the brain-blood barrier and that many cancer cells are often not significantly affected by the immune system.

Thus, there has existed a need to develop a novel therapeutic agent and method which can be targeted specifically to tumor cells without harming non-tumor cells, and has no immunotoxicity so that repeated administrations of the therapeutic agent are possible.

MSCs are multipotent bone marrow stromal cells which can differentiate into mesodermal lineage cells such as osteocytes, chondrocytes, adipocytes and myocytes. MSCs can also be easily proliferated with maintaining their undifferentiated states which are suitable for carrying anti-cancer agents against glioblastoma owing to their tropism toward tumor cells.

Suicidal genes are capable of converting a non-toxic prodrug to corresponding anti-cancer drug. For example, CD can convert 5-fluorocytosine (5-FC) to cytotoxic anticancer agent, 5-fluorouracil (5-FU), and secreted 5-FU kills neighboring cells, which is called a "bystander effect".

The direct administration of 5-FU causes cytotoxicity and leads to adverse side effects, but the combined use of a suicidal gene and 5-FC in a specific way makes it possible to generate 5-FU selectively around tumor cells (Bourbeau et al., The Journal of Gene Medicine, 6, 1320-1332 (2004)).
WO99/37332 discloses bone cancer therapy using a homing bone marrow cell line including a suicide gene.

The present inventors have endeavored to develop a safe and effective anticancer agent using MSCs; and have found that a brain tumor can be efficiently cured when MSCs expressing a suicide gene are transplanted into the brain of a brain cancer animal model, followed by administering a prodrug of an anticancer agent.

### SUMMARY OF THE INVENTION

Accordingly, it is an object of the present invention to provide a means for treating a cancer, which exhibit a high targeting efficiency to cancer tissues, non-toxicity toward normal cells, and no immunotoxicity, which allows repeated administration of the composition.

In accordance with one aspect of the present invention, a mesenchymal stem cell expressing a suicide gene is used for the manufacture of a medicament for treating a cancer.

### BRIEF DESCRIPTION OF THE DRAWINGS

The above will become apparent from the following description of the invention, when taken in conjunction with the accompanying drawings, which respectively show:
Fig. 1: light microscopic photographs of mesenchymal stem cells (MSCs) isolated from human bone marrow after cultivating for 1, 2, 3, and 7 days *in vitro*;
Fig. 2: photographs showing multi-potentials of MSCs (A: adipocytes stained with oil red-O; B: chondrocytes stained with alcian blue; C: alkaline phosphatase activity in osteocytes; and D: osteocytes stained with von Kossa);
Fig. 3: the construction of a retroviral vector comprising the cytosine deaminase (CD) gene of *E. coli* as a suicide gene,;
Figs. 4A to 4C: graphs and photograph showing expression of CD in 293T cells (A: activity of expressed CD which converts [³H] cytosine to [³H] uracil; B: cell death of 293T cells expressing the CD, depending on the conc. of 5-FC which is the prodrug of 5-FU; and C: light microscopic photographs of 293T and 293T/CD cells cultivated in the presence of 1,000 µM 5-FC);
Figs. 5A and 5B: photographs and a graph showing the bystander effects of 293T/CD cells (Fig. 5A: phase-contrast microscopic photographs of 293T and 293T/CD cells after co-culture with C6/LacZ glioma cells in the presence of 1,000 µM 5-FC, and light microscopic photographs taken after staining with X-gal (Magnification: x100 each); and Fig. 5B: the graph showing β-galactosidase (β-gal) activity in the 293T and 293T/CD cells with the conc. of 5-FC);
Figs. 6A and 6B: photographs and a graph showing the cell death of MSCs and the MSCs transfected with a retroviral vector comprising a CD gene (MSC/CD), upon treatment with 5-FC (Fig. 6A: light microscopic photographs; and Fig. 6B: the graph of MTT analysis results);
Figs. 7A to 7C: photographs and a graph showing the bystander effects of the MSC/CD cells (Fig. 7A: phase-contrast microscopic photographs of MSCs and MSC/CD cells after co-culture with C6/LacZ glioma cells in the presence of 1,000 µM 5-FC and light microscopic photographs taken after staining with X-gal (Magnification: x100 each); Fig. 7B: the graph showing the survival rate of C6/LacZ cells in the β-gal assay; and Fig. 7C: dissect microscopic and digital microscopic photographs of whole wells);
Figs. 8A and 8B: results of HPLC analyses showing the conversion of prodrug 5-FC to 5-FU in the MSCs and MSC/CD cells (Fig. 8A: HPLC chromatograms of MSCs and MSC/CD cells; and Fig. 8B: a graph showing the amount of detected 5-FU);
Fig. 9: a graph showing the cell death rate of C6, U373 and U87 glioma cell lines with the conc. of 5-FU;
Fig. 10: results showing tropism of MSCs for glioma (A: schematic diagram showing the migration of MSC/LacZ transplanted in one hemisphere to the contralateral glioma-bearing hemisphere; B: microscopic photograph of a brain tissue section showing the migration of MSC/LacZ to the glioma-bearing hemisphere; and C and D: microscopic photographs of 100 and 200 magnifications, respectively, showing the distribution of MSC/lacZ cells at the boundary of glioma);

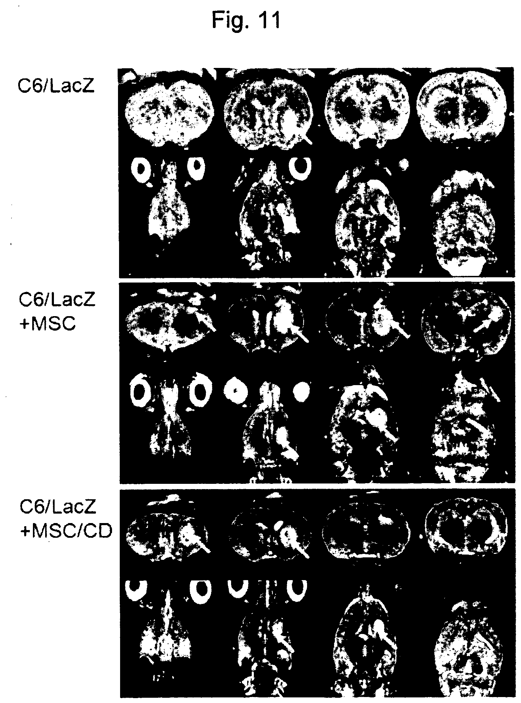

Fig. 11: MRI images showing the anticancer effects of MSCs expressing CDs; and
Fig. 12: results of the X-gal staining of brain sections from brain tumor rat models, which show the anticancer effects of MSCs expressing CDs.

### DETAILED DESCRIPTION OF THE INVENTION

The suicide gene used in the present invention is cytosine deaminase (CD). CD converts non-toxic 5-fluorocytosine (5-FC) to toxic 5-fluorouracil (5-FU). CD gene is preferable for use in the gene therapy because 5-FU exhibits a strong bystander effect.

The suicide gene may be introduced into a mesenchymal stem cell (MSC) by employing a viral vector, preferably, a retroviral vector, comprising the gene in accordance with any known method for introducing a gene into a cell. For instance, the suicide gene can be introduced into the mesenchymal stem cell by introducing it into a retroviral vector to obtain an expression vector, transfecting a packaging cell with the expression vector, culturing the transfected cell under an appropriate culture condition, filtering the culture medium to obtain a retroviral solution, and transfecting MSCs with the retroviral solution. Then, MSCs which continuously express the suicide gene can be obtained by using a selection marker contained in the retroviral vector.

The mesenchymal stem cell expressing a suicide gene may be mass produced *in vitro* by introducing the suicide gene into stem cells and selecting, and amplifying the resulting stem cells under proper conditions; or by sufficiently proliferating stem cells, introducing the suicide gene into the proliferated stem cells and harvesting the resulting stem cells.

The stem cells which may be used in the present invention can be isolated from the bone marrow, peripheral blood or cord blood of any mammal including human, preferably from the human bone marrow.

The medicament comprising mesenchymal stem cells expressing a suicide gene is useful in the treatment of a cancer. The cancers include brain cancer, breast cancer, liver cancer, pancreas cancer, colorectal cancer, and lung cancer.

The medicament may further comprise pharmaceutically acceptable excipients, carriers, or diluents. Preferably, it may be formulated into an injection formulation suitable for injecting into a tissue or organ.

The medicament may additionally include lubricating agents, flavoring agents, emulsifiers, preservatives and the like.

The medicament can be injected into the patient's body according to the conventional methods well known in the art such as the clinical method disclosed by Bjorklund and Stenevi (Brain Res., 177, 555-560 (1979) and Lindvall et al. (Arch. Neurol., 46, 615-31(1989)).

The unit dose of the mesenchymal stem cells of the present invention to be actually administered ought to be determined in light of various relevant factors including the disease to be treated, the severity of the patient's symptom, the chosen route of administration, and the age, sex and body weight of the individual patient.

Moreover, illustrated is a kit for treating a cancer comprising an expression vector comprising a suicide gene, a mesenchymal stem cell and a prodrug of an anticancer agent.

In the kit, the expression vector comprising the suicide gene and the mesenchymal stem cell may be provided separately or provided in the form of a mesenchymal stem cell transfected with the expression vector comprising the suicide gene or transduced with viruses expressing the suicide gene. The expression vector is preferably prepared by introducing the suicide gene into a viral vector, preferably, a retroviral vector.

Also illustrated is a method for treating a subject suffering from a cancer, which comprises administering a therapeutically effective amount of mesenchymal stem cells expressing a suicide gene to the subject, followed by the administration of a prodrug of an anticancer agent.

Immune rejections can be minimized in gene therapy using the medicament when the mesenchymal stem cells are obtained from the patient's own bone marrow or from the bone marrow of others having the same HLA (human leukocyte antigen) type as the patient. Accordingly, the medicament can be repeatedly administered for thoroughly preventing the relapse of the cancer. Further, the use of the mesenchymal stem cells in gene therapy have advantages in that the mesenchymal stem cells can be easily cultured without using an oncogene and obtained in a large amount sufficient for transplantation to the patient.

Moreover, the medicament can be applied for the treatment of a cancer hiding from immune surveillance because the effect thereof does not depend on improvement of immune responses.

Accordingly, the medicament can be used alone or in combination with other therapy for the treatment of a cancer, especially an intractable cancer.

Upon injection, the mesenchymal stem cells in the medicament exhibit tropism toward cancer tissues. Accordingly, adverse side effects to the normal cells can be minimized by specifically delivering the mesenchymal stem cells expressing a suicide gene to cancer tissues and, then, administering a prodrug of an anticancer to be activated by the suicide gene so that the anticancer agent is generated only around the cancer tissues. Further, the mesenchymal stem cells expressing a suicide gene exhibits bystander effects on surrounding cancer cells wherein the suicide gene is not directly introduced, thereby increasing its anticancer effect.

The following Examples are intended to further illustrate the present invention

Further, percentages given below for solid in solid mixture, liquid in liquid, and solid in liquid are on a wt/wt, vol/vol and wt/vol basis, respectively, and all the reactions were carried out at room temperature, unless specifically indicated otherwise.

### Example 1: Isolation and culture of human mesenchymal stem cells (hMSCs)

### (Step 1) Extraction of bone marrow and isolation of mesenchymal stem cells

4 ml of HISTOPAQUE 1077 (Sigma, U.S.A.) and 4 ml of bone marrow obtained from Bone marrow bank (Korean Marrow Donor Program, KMDP) were added to a sterilized 15 ml test-tube. After centrifugation at 400 x g for 30 minutes, 0.5 ml of the buffy coat in the interphase was collected and transferred into a test-tube containing 10 ml of sterilized phosphate buffered saline. The resulting suspension was centrifuged at 250 x g for 10 minutes to remove the supernatant and 10 ml of phosphate buffer was added thereto to obtain a suspension, which was centrifuged at 250 x g for 10 minutes. The above procedure was repeated twice and DMEM medium (Gibco, U.S.A.) containing
10 % FBS (Gibco) was added to the resulting precipitate. A portion of the resulting solution corresponding to 1 x 10⁷ cells was placed in a 100 mm dish and incubated at 37°C for 4 hours while supplying 5 % CO₂ and 95 % air. The supernatant was then removed and a new medium was added to continue culturing.

### (Step 2) Culture and subculture of hMSCs

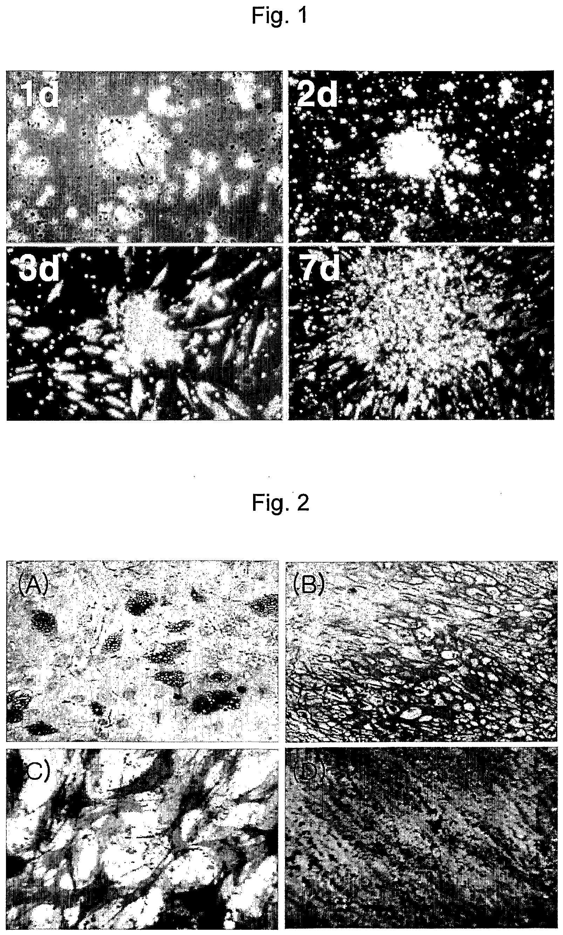

The hMSCs obtained in Step 1 were incubated using an MSC medium (10 % FBS (Gibco) + 10 ng/mℓ bFGF (Sigma) + 1 % penicillin/streptomycin (Gibco) + 89 % DMEM (Gibco)) in a CO₂ incubator kept at 37°C. Serial incubations were carried out while changing the medium at an interval of 2 days. When the cells reached to 80 % confluence, the cells were collected using 0.25 % trypsin/0.1 mM EDTA (Gibco) and diluted 20 folds with the medium, and then, subcultured in the new dishes. The rest of cells thus obtained were kept frozen in a medium containing 10 % DMSO (Sigma). Fig. 1 shows light microscopic photographs of mesenchymal stem cells isolated from human bone marrow after cultivating for 1, 2, 3, and 7 days *in vitro.*

### (Step 3) Multiple differentiation potentials of hMSCs

*In vitro* differentiation potentials of hMSCs into adipocytes, chondrocytes and osteocytes were examined as follows.

### (1) Adipogenic differentiation of hMSCs

hMSCs were cultured in the MSC medium, followed by culturing in an adipogenic differentiation induction medium (DMEM medium (Gibco) supplemented with 1 µM dexamethasone (Sigma), 0.5 mM methylisobutylxanthine (Sigma), 10 *µ*g/mℓ of insulin (Gibco), 100 nM indomethacin (Sigma) and 10 % FBS (Gibco)) for 48 hours. The resulting mixture was subsequently incubated in an adipogenic maintenance medium (DMEM medium containing 10 *µ*g/mℓ of insulin and 10 % FBS) for 1 week and stained with oil red O. As shown in Fig. 2, adipocytes stained with oil red O is pronounced inside the cells (Fig. 2, A). This result suggests that hMSCs can successfully differentiate into adipocytes.

### (2) Chondrogenic differentiation of hMSCs

hMSCs were cultured in the MSC medium. 2 x 10⁵ of the cells were collected using trypsin, centrifuged, and then, re-incubated in 0.5 mℓ of a serum-free chondrogenic differentiation induction medium (50 mℓ of high-glucose DMEM (Gibco) containing 0.5 mℓ of 100 x ITS (0.5 mg/mℓ of bovine insulin, 0.5 mg/mℓ of human transferrin, 0.5 *µ*g/mℓ of sodium selenate (Sigma) and 10 ng/mℓ of TGF-β1 (Sigma)) for 3 weeks while replacing the medium every 3 days. Then the cell masses were fixed with 4 % paraformaldehyde, sectioned using a microtome, and then, stained with alcian blue. As shown in Fig. 2, the extracellular cartilage matrix was stained blue and the presence of chondrocytes in cartilage lacunae was observed (Fig. 2, B). These results suggest that the hMSCs differentiated into chondrocytes.

### (3) Osteogenic differentiation of hMSCs

hMSCs were incubated in the MSC medium, followed by culturing in 0.5 mℓ of an osteogenic differentiation induction medium (DMEM supplemented with 10 mM β-glycerol phosphate (Sigma), 0.2 mM ascorvate-2-phosphate (Sigma), 10 nM dexamethasone (Sigma) and 10 % FBS (Gibco)) for 2 weeks while replacing the medium every 3 days. Then the cells were fixed with 4 % paraformaldehyde, and stained for alkaline phosphatase and with von Kossa method, respectively. As shown in Fig. 2, the increase of the alkaline phosphatase activity and the extracellular accumulation of calcium minerals in the form of hydroxyapatite suggest that the hMSCs differentiated into osteocytes (Fig. 2, C and D).

### Example 2: Construction of a retroviral vector expressing cytosine deaminase

### (Step 1) Cloning of cytosine deaminase

Cytosine deaminase (CD) gene was cloned by PCR reaction using genomic DNA of *E*. *coli* K12 MG1655 (ATCC 700926, Korea Research Institute of Bioscience and Biotechnology) as a template. PCR was carried out using oligonucleotides CD-F (5'-GAA TTC AGG CTA GCA ATG TCT CGA ATA ACG CTT TAC AAA C-3': SEQ ID NO: 1) and CD-R (5'-GGA TTC TCT AGC TGG CAG ACA GCC GC-3'; SEQ ID NO: 2) under the condition of 5 minutes at 94°C; 27 cycles of 30 seconds at 94°C, 40 seconds at 60°C and 1 minute at 72°C; 7 minutes at 72°C. The PCR product was cloned using pGEM-T Easy vector cloning kit (Promega) and vector pGEM-T-CD containing the CD gene was isolated by blue-white colony selection using X-gal and IPTG. Through sequence analysis of vector pGEM-T-CD using oligonucleotides of SEQ ID NO: 3 (5'-CAT ACG ATT TAG GTG ACA CTA TAG-3') and SEQ ID NO: 4 (5'-ACC GGG AAA CAC CTA TTG TG-3'), the CD gene (gi298594) was verified.

### (Step 2) Construction of a retroviral vector expressing cytosine deaminase

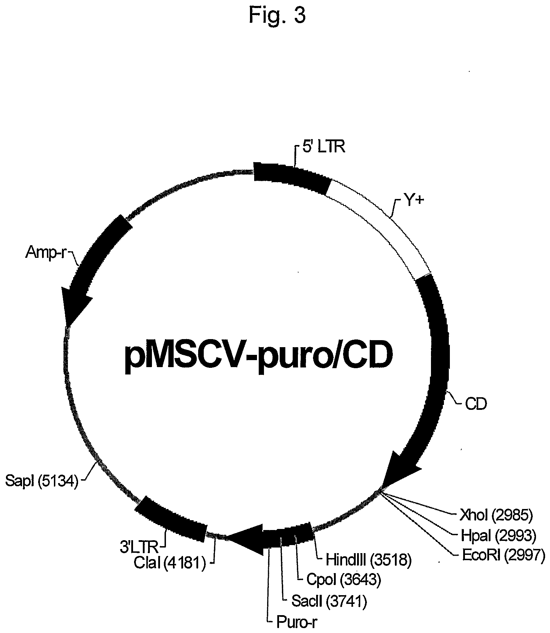

The CD cDNA was isolated from vector pGEM-T-CD with *Eco*RI (Roche, Germany) and *Not*I (Roche), and inserted into the *Eco*RI and *Not*I sites of vector pcDNA3.1 (Clontech, U.S.A.) using T4 DNA ligase (Roche). *E. coli* DH5α was transformed with the resulting vector, and the resulting transformant was cultured and selected in LB plate with 50 *µ*g/mℓ of ampicillin to obtain pcDNA3.1/CD. The CD cDNA was isolated from pcDNA3.1/CD with *Bam*HI (Roche), and inserted into the *Bgl*II (Roche) site of the retroviral vector pMSCV-puro (Clontech) with T4 DNA ligase (Roche). The resulting construct designated pMSCV-puro/CD is shown in Fig. 3.

### (Step 3) Preparation of retrovirus

pMSCV-puro/CD vector was transfected into a retroviral packaging cell line, PA317 (ATCC CRL-9078) or PG13 (ATCC CRL-10686) with the calcium phosphate-coprecipitation method [Jordan, Nucleic Acid Research, 24, 569-601(1996)], and the cells were cultured at 37°C, 5 % CO₂. After 48 hours, the culture solution was collected and filtered with a 0.45 µm nylon membrane to obtain retrovirus solution. The retrovirus solution was kept at -70°C until use.

### Example 3: Verification of CD expression in 293 T cell

### (Step 1) Quantification of cytosine deaminase activity

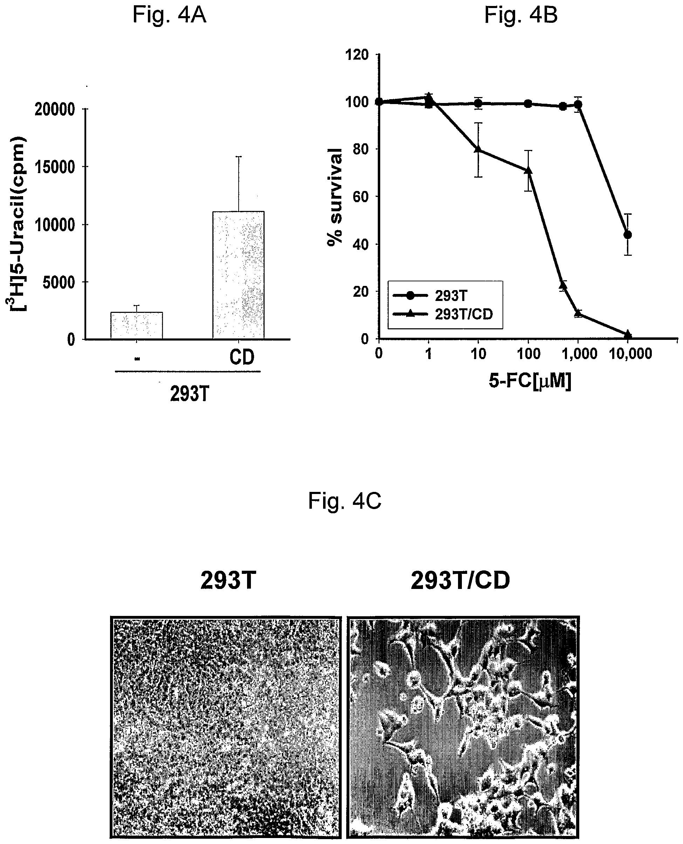

pMSCV-puro/CD was transfected into 293T cell (ATCC CRL-11268) according to the calcium phosphate-coprecipitation method. After 48h of transfection, the cells were split into 1:10 in the selection medium containing puromycin (4 *µ*g/mℓ, Sigma, U.S.A.) for 2 weeks. The grown cells were harvested in phosphate buffered saline and lysed by repeating 5 times of freezing in dry-ice in ethanol for 2 minutes and thawing at 37°C for 5 minutes. A supernatant was obtained by centrifuging the cell lysate at 12,000 rpm for 5 minutes at 4°C, and protein concentration was quantified by Bradford method (Anal Biochem, 72: 248-254, 1976). Ten *µ*g proteins in 10 *µ*ℓ were mixed with *5 µ*ℓ of 3 mM [6-³H]cytosine (0.14 mCi/mmol, Moravek, USA) and incubated at 37°C for 1 h. The reaction was terminated by adding 345 *µ*ℓ of 1 M acetic acid. The mixture was eluted with a SCX Bond elute column (Varian, U.S.A) rinsed by 1 mℓ of 1 M acetic acid and the produced [6-³H]uracil was determined with a liquid scintillation counter. 293T cells transformed with pMSCV-Puro were used as a negative control in this experiment. As shown in Fig. 4A, the experimental group in 293T transformed pMSCV-puro/CD was higher 4 times than the negative control group. This result indicates that pMSCV-puro/CD was functionally expressed.

### (Step 2) Suicide effects of CD gene in 293T cells

Normal 293T cells and 293T/CD cells transformed with pMSCV-puro/CD prepared in Step 1 were plated at a density of 2,000 cells/well in a 24-well plate containing DMEM with 10 % fetal bovine serum. After 24 hours, 5-FC was added to the cells at various concentrations (0-10 mM). The media was replaced with fresh medium containing 5-FC every 2 days for 1 week. As shown in Fig. 4C, the viability of 293T/CD were reduced in the presence of 1,000 µM 5-FC as determined by phase contrast microscopy. The viability was estimated by measuring mitochondria NADPH activity by 3-(4,5-dimethyl-thiazol-2-yl)-2,5-diphenyltetrazolium bromide (MTT) assay. The cells were incubated in 200 *µ*ℓ of 25 mg/mℓ MTT (Sigma, U.S.A) in PBS for 4 hours. The solution was replaced with 200 *µ*ℓ of absolute dimethyl sulfoxide (DMSO, Sigma). After incubated at room temperature for 15 minutes, the absorbance at 540 nm was measured with an ELISA reader (Molecular Probe, U.S.A). The data in Fig. 4B are presented as averages ± S.E. with respect to that of the cells grown in the absence of 5-FC from 3 independent experiments. The cell death of the 293T/CD was detected from the 10 µM of 5-FC with IC₅₀ of 296 µM. In contrast, 293T cells did not show cell death in the presence of 1,000 µM of 5-FC.

### (Step 3) In vitro bystander effects of 293T/CD cells

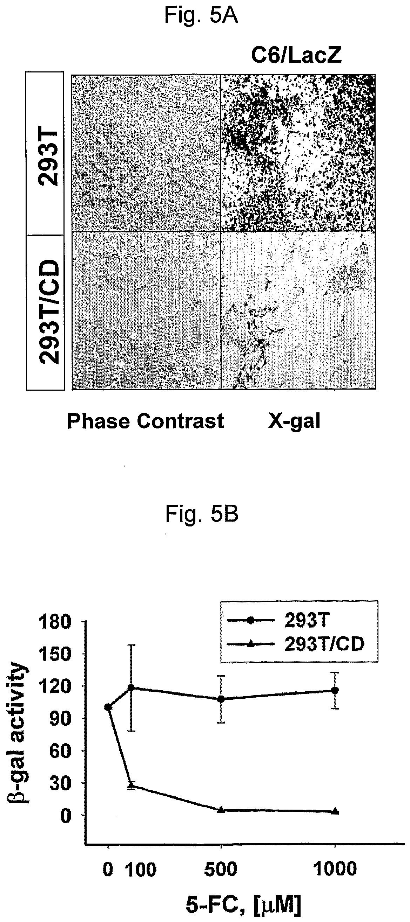

In 6-well plates, 3X10³/well of 293T/CD and 293T cells were respectively co-cultured with 3X10³ C6/LacZ glioma cells (ATCC CRL2199). C6/LacZ cells were originally obtained by transforming C6 glioma cells with the *E. coli* LacZ gene and used to determine the bystander effect of 293T/CD cells. After 24 hours, 5-FC was added to the cells at indicated concentrations. The media was replaced with fresh medium containing 5-FC every 2 days for 1 week. The culture was subject to X-gal staining or β-galactosidase (β-gal) assay. For X-gal staining, the cells were fixed with 0.00625 % glutaraldehyde (Merck, U.S.A.) for 10 min, rinsed 3 times with PBS and incubated in 2 mM MgCl₂, 5 mM potassium ferrocyanide/potassium ferricyanide, and 1 mg/mℓ X-gal (Koma, Korea) for 8 hours. As shown in Fig. 5A, C6/LacZ cells that were co-cultured with parental 293T cells were increased in numbers and stained blue with X-gal. In contrast, when co-cultured with 293T/CD, C6/LacZ cells were died in the presence of 1,000 µM of 5-FC due to the bystander effect of 293T/CD.

The survival of C6/LacZ cells were further confirmed with β-gal assay which measures the remnant enzyme activity of LacZ gene product, P-galactosidase. The cells were harvested and lysed in 1X passive lysis buffer (Promega, U.S.A.). The 10 *µ*g protein of cell lysates was incubated in 300 *µ*ℓ of 0.88 mg/mℓ ONPG (Sigma), 1 mM MgCl₂ and 0.1M phosphate buffer (pH 7.4) at 37°C about 30 minutes. The reaction was stopped by adding 500 *µ*ℓ of 1 M Na₂CO₃ and absorbance of the mixture were determined at 420 nm. Fig. 5B presents the relative ratios with respect to that of untreated cells. Consistently with the result shown in Fig. 5A, C6/LacZ co-cultured with 293T did not altered by 5-FC in β-gal assay. In contrast, β-galactosidase activity of C6/LacZ cells was reduced by co-cultivation with 293T/CD in the presence of 100-1,000 µM 5-FC. The data indicate that co-culture with 293T/CD induces cell death of neighboring C6/LacZ cells due to bystander effects in the presence of 5-FC.

### Example 4: Preparation of hMSC expressing cytosine deaminase

### (Step 1) Introduction of CD genes into hMSC

CD-expressing retrovirus was used to deliver the CD gene into MSCs. MSCs obtained in Step 1 of Example 1 were grown in growth medium (DMEM containing 10 % FBS, 100 units/mℓ penicillin and 100 *µ*g/mℓ streptomycin) at 37°C and 5 % CO₂ until 70 % confluence. CD-expressing retrovirus prepared in Example 2 was added to the culture with 1:1 dilution in the growth medium in the presence of 8 *µ*g/mℓ polybrene (Sigma) for 8 hours and in the fresh growth medium for 16 hours. After the retroviral transduction was repeated 2 more times, the cells were split into 1:3 in growth medium containing 4 *µ*g/mℓ puromycin (Sigma) for 14 days. The survived cells were pooled, and used in the subsequent experiments.

### (Step 2) Suicide effects of CD gene in MSC/CD cells

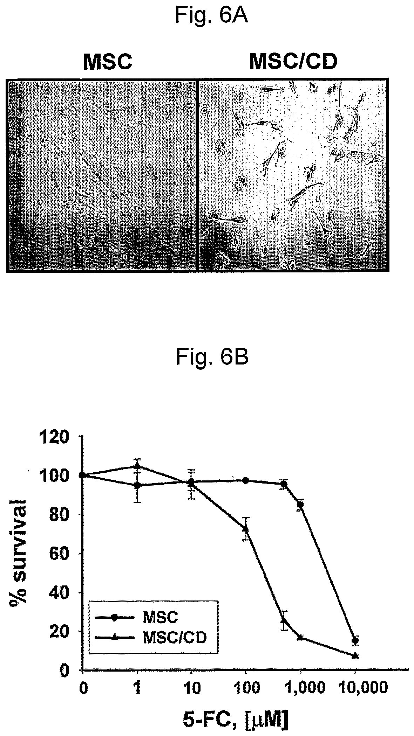

MSC/CD or MSC cells were plated in growth medium at a density of 5,000 cells/well in a 6-well plate one day before exposure to 5-FC. The medium was replaced every 2 days with fresh growth medium containing 0-10 mM 5-FC for 2 weeks. As shown in Fig. 6A, MSC/CD cells almost died after treatment with 1,000 µM 5-FC for 2 weeks. The viability was estimated by measuring mitochondria NADPH activity using MTT assay as mentioned above with 293T/CD cells. The data are presented as averages ± S.E with respect to that of the untreated cells from 3 independent experiments (Fig. 6B). The cell death of MSCs/CD was detected from the 100 µM of 5-FC with IC₅₀ = 332 µM. In contrast, MSCs did not show cell death up to 1,000 µM of 5-FC.

### (Step 3) In vitro bystander effects of MSC expressing CD genes

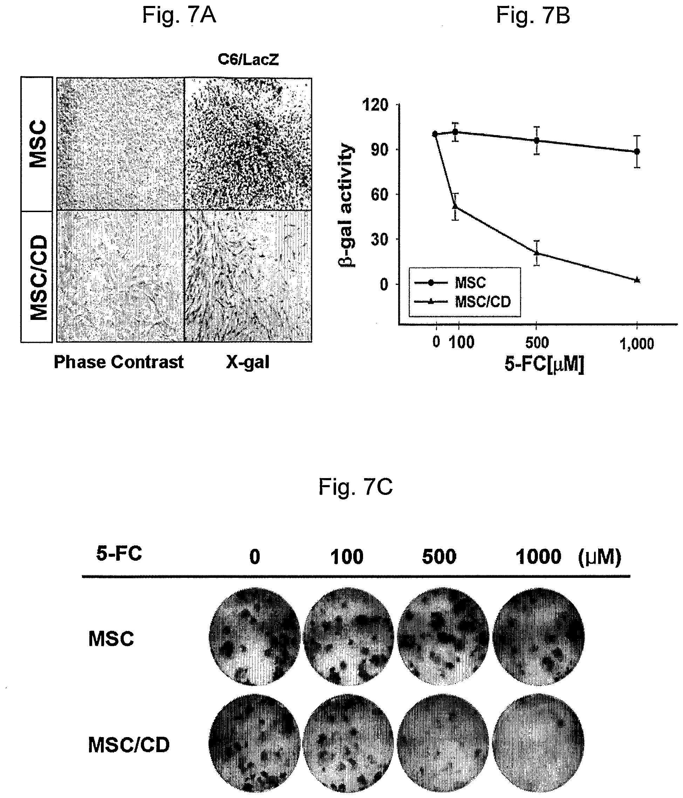

In 6-well plates, 1X10⁴/well of MSC/CD and MSC cells were respectively co-cultured with 3X10³ C6/LacZ glioma cells in growth medium. After 24 hours, 5-FC was added to the cells to final concentrations of 1-1,000 µM and medium was replaced every 2 days for 1 week. The remained cells were subject to X-gal staining (Figs. 7A and 7C) or β-galactosidase assay (Fig. 7B) as described above with 293T/CD cells. In the presence of 1000 µM of 5-FC, MSC/CD cells induced cell death of C6/LacZ whereas MSC did not alter reduced proliferation of C6/LacZ cells (Figs. 7A and 7C). Therefore, colony formation frequency of C6/LacZ cells was reduced upon co-cultivation with MSC/CD in a dose dependent manner (Fig. 7C). The data indicate that MSC/CD cells exert the bystander effect on C6/LacZ cells in the presence of 5-FC.

The survival of C6/LacZ cells was further verified by β-gal assay, by which the survived cells were indirectly quantified. Assays were carried out in a similar way as described above with 293T/CD cells using 10 *µ*g proteins of cell lysates. Absorbance at 420 nm was presented as a relative ratio with respect to that of untreated cells. As shown in Fig. 7B, β-galactosidase activity was dramatically reduced in C6/LacZ cells in a concentration dependent manner when the cells were co-cultured with MSC/CD cells. In contrast, β-galactosidase activity was not altered in C6/LacZ cells that were co-cultured with normal MSCs. Again, MSC/CD cells exert bystander effects on C6/LacZ cells in the presence of 5-FC.

### (Step 4) Verification of conversion of 5-FC to 5-FU by MSC/CD

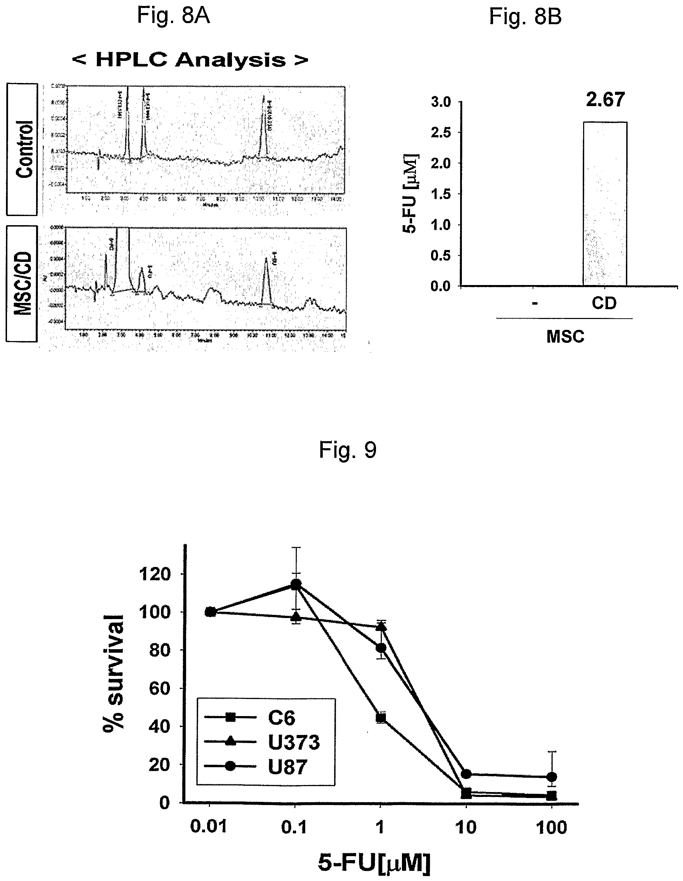

MSC and MSC/CD cells were plated at a density of 1X10⁴ cells/well in 12-well plates and incubated in the presence of 1,000 µM 5-FC for 3 days. The 50 *µ*ℓ of medium were extracted by 500 *µ*ℓ of ethyl acetate:isopropanol:0.5mol/L acetic acid (84:15:1(v/v/v)) with 0.3 *µ*g of 5-bromouracil (5-BU, Aldrich, U.S.A.). After the organic fraction was dried with a vacuum evaporator at 4 °C for 90 minutes, the pellet was reconstituted in 500 *µ*ℓ of the mixture of H₂O:methanol (4:1). HPLC was performed using Xterra TM RP18 5 µm C-18 column (4.6x150 mm, Waters, U.S.A). Elution was carried out isocratically at a flow rate of 0.6 mℓ/min with a isocratic mobile phase consisting in 40 mM KH₂PO₄ and adjusted to pH 7.0 with 10 % KOH. 5-FC and 5-FU were eluted in 3.1 min and 3.9 min. 5-BU, an internal standard, was eluted in 10.2 min (Fig. 8A). As shown in Fig. 8B, MSC/CD converted 1,000 µM 5-FC to 2.67 µM 5-FU whereas MSC could not produce any 5-FU. The results indicated conversion of 5-FC to 5-FU was specific to CD gene.

### Example 5: Validation of cytotoxic effect of 5-FU produced by MSC/CD on various glioma cell lines

To verify that 5-FU produced by MSC/CD is sufficient to induce cell death in various glioma cells, C6 (KCLB No. 10107), U373 (KCLB No. 30017), U87 (KCLB No. 30014) cells were freshly obtained from KCLB (Korean cell line bank) and plated in 96-well plates in growth medium at a density of 100 cells/well, 100 cells/well, and 500 cells/well, respectively. After 24 hours, 5-FU was added to the cells at 0.01-100 µM. After 1 week, cell viability was estimated by MTT assay as described above. As shown in Fig. 9, 5-FU induced cell death in all glioma cell lines with IC₅₀ of 0.93, 5.34, and 5.65 µM for C6, U373 and U87, respectively, and reached plateau at 10 µM. The data indicate that concentrations of 5-FU as a product of conversion from 5-FC by MSC/CD is sufficient to induce cell death in various glioma cells.

### Example 6: In vivo tropism of MSC to brain tumor

### (Step 1) Transplantation

Brain tumor was induced by transplantation of C6/LacZ cells into the brain of adult male Sprague-Dawley albino rats (Samtaco, Korea) weighing about 250 g. Briefly, the rats were anesthetized by intraperitoneal injection of 400 mg/kg chloral hydrate (Sigma). After the fur at the incision region was removed, the animals were fixed to a stereotaxic frame (Koepfer, Germany). The vertex was sterilized with 70 % ethanol and the 1 cm incision was made. A hole was made by drilling the dura at positions of bregma -0.5, ML + 3.0 and DV + 4.0, 5 x 10⁵ U373 cells in 3 µl PBS were injected at a rate of 0.2 µl/min using a Hamilton syringe. After the surgery, the incision was sewn and the animals were placed back to the cage. Four days later, 2 x 10⁵ cells of MSC/LacZ cells were transplanted to a contralaterally at the position of bregma -0.5, ML - 3.0 and DV + 4.0, 5 µl of PBS containing was inoculated. Twenty minutes after injection, the syringe was removed. The incision was sutured. The rat's brain was extracted after the glioma was transplanted for 2 weeks.

### (Step 2) Preparation of tissue slice

Two weeks after the 2^{nd} transplantation, rats were anesthetized by injecting 400 mg/kg of chloral hydrate (Sigma). The rats were perfused with PBS and then with 0.1 M paraformaldehyde in PBS (pH 7.4). The brain was extracted and kept at 4 °C in 0.1 M paraformaldehyde in PBS (pH 7.4) for 16 hours to post-fix, and then in 30 % sucrose for 24 hours. The sections were made using a sliding microtome with a thickness of 35 µm and mounted to silane-coated slides (Muto Purew Chemicals, Japan) and stored at 4 °C in PBS till use.

### (Step 3) X-gal staining

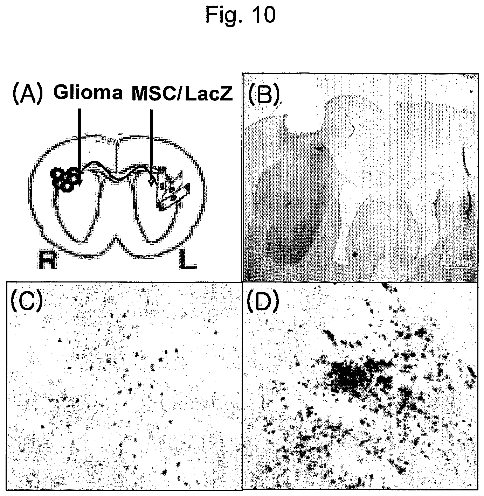

The brain sections mounted on a slide were rinsed 3 times with PBS for 10 minutes and incubated in 2 mM MgCl₂, 5 mM potassium ferrocyanide/potassium ferricyanide, and 1 mg/mℓ X-gal (Koma) for 8 hours. To enhance the contrast, sections were counterstained with eosin, dehydrated, and covered with Balsam. As shown in Fig. 10B, X-gal+ blue MSC/LacZ cells migrated from one hemisphere to the other hemisphere where glioma cells were firstly transplanted. It is notable that some cells remained in the needle track where the MSC/LacZ cells were transplanted. In higher magnification of the microscopy, the MSC/LacZ cells infiltrated to the tumor mass (Fig. 10C) as well as along the tumor edge (Fig. 10D). The data indicate that MSCs have high potential to migrate to tumor sites and to be used as vehicles to deliver suicide genes to brain tumor.

### Example 7: Anti-tumor effects of MSC/CD in a brain tumor animal model

### (Step 1) Transplantation

As described in Step 1 of Example 6, adult Sprague-Dawley albino rats (about 250 g) were anesthetized. At positions of bregma -0.5, ML + 3.0 and DV + 4.0, 5 x 10⁴ C6/LacZ cells were transplanted with 5 x 10⁵ each of MSC/CD or MSC in 5 µl PBS at a rate of 0.5 µl/min using a Hamilton syringe. From next day the rats were intraperitoneally administered with 500 mg/kg/day of 5-FC for 14 days.

### (Step 2) Measuring of anti-tumor effect by MRI

Magnetic resonance imaging (MRI) was performed at 2 weeks after the transplantation of the cells to estimate intracerebral tumor volume. Animal were anesthetized by intraperitoneal injection of 400 µl chloral hydrate and placed on a surface coil specially made for rat study. MRI scanning was performed with a 3.0 Tesla MRI system (Magnum 3.0; Medinus Inc., Korea) and a birdcage RF coil (diameter: 30 cm). The repetition time (TR) and echo time (TE) were 1400 ms and 60 ms, respectively. The slice thickness was 1.5 mm with no slice gaps. MRI analysis indicated that transplantation with MSCs/CD decreased tumor volume compared to the animals with PBS or untreated MSCs.

### (Step 3) Verification of correlation of MRI with histological methods

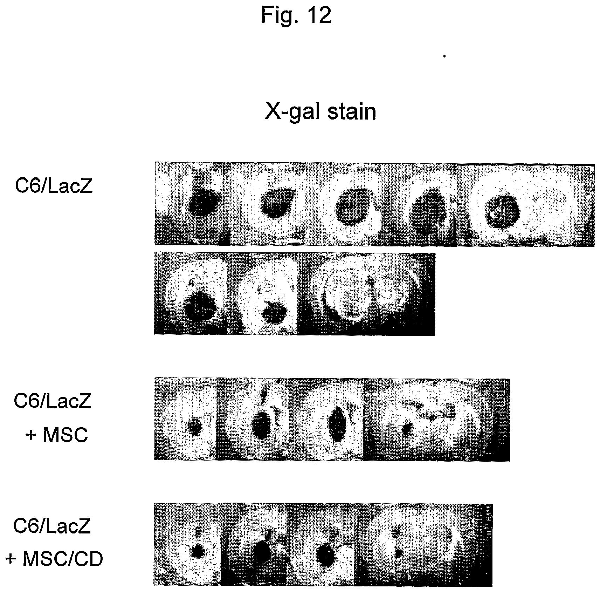

To further confirm the reduction of brain tumor by MSC/CD, the brain of the same animals that were used for MRI was extracted as described above except that the section was 100 µm thick and subject to X-gal staining. The tumor size was smaller in animals that received MSC/CD compared to animals treated with PBS or with MSC (Fig. 12). The data obtained both with MRI and X-gal staining consistently indicated that the brain tumors were reduced in size with transplantation of MSCs/CD and administration of 5-FC.

## Claims

1. Use of a mesenchymal stem cell expressing a suicide gene for the manufacture of a medicament for treating a cancer,
wherein the suicide gene is a gene encoding cytosine deaminase,
wherein cytosine deaminase expressed from the suicide gene converts the 5-fluorocytosine to 5-fluorouracil,
wherein the mesenchymal stem cell is a human mesenchymal stem cell isolated from bone marrow, peripheral blood or cord blood, and
wherein the cancer is selected from brain cancer, breast cancer, liver cancer, pancrease cancer, colorectal cancer and lung cancer.

2. The use of claim 1,
wherein the medicament is manufactured for administration prior to the administration of 5-fluorocytosine.

3. A mesenchymal stem cell expressing a suicide gene for use in treating a cancer in a subject,
wherein the suicide gene is a gene encoding cytosine deaminase,
wherein cytosine deaminase expressed from the suicide gene converts the 5-fluorocytosine to 5-fluorouracil,
wherein the mesenchymal stem cell is a human mesenchymal stem cell isolated from bone marrow, peripheral blood or cord blood, and
wherein the cancer is selected from brain cancer, breast cancer, liver cancer, pancrease cancer, colorectal cancer and lung cancer.

4. The mesenchymal stem cell for use according to claim 10,
wherein the mesenchymal stem cell is for administration prior to administration of 5-fluorocytosine.

## Patentansprüche

1. Verwendung eines mesenchymalen Stammzelle, welche ein Suizid-Gen exprimiert zur Herstellung eines Medikaments zur Behandlung einer Krebsart,
wobei das Suizid-Gen ein Gen ist, das Cytosindeaminase kodiert,
wobei die Cytosindeaminase, exprimiert aus dem Suizid-Gen 5-Fluorocytosin in 5-Fluorouracil konvertiert,
wobei die mesenchymale Stammzelle eine menschliche mesenchymale Stammzelle ist, isoliert aus Knochenmark, peripheralem Blut oder Nabelschnurblut, und
wobei der Krebs ausgewählt ist aus Gehirnkrebs, Brustkrebs, Leberkrebs, Bauchspeicheldrüsenkrebs, Darmkrebs und Lungenkrebs.

2. Die Verwendung von Anspruch 1,
wobei das Medikament hergestellt wird zur Verabreichung vor der Verabreichung von 5-Fluorocytosin.

3. Eine mesenchymale Stammzelle, welche ein Suizid-Gen exprimiert, zur Verwendung in der Behandlung einer Krebsart in einem Subjekt,
wobei das Suizid-Gen ein Gen ist, das Cytosindeaminase kodiert,
wobei Cytosindeaminase, exprimiert aus dem Suizid-Gen, 5-Fluorocytosin in 5-Fluorouracil konvergiert,
wobei die mesenchymale Stammzelle eine menschliche mesenchymale Stammzelle ist, isoliert ist aus Knochenmark, peripheralem Blut oder Nabelschnurblut und
wobei der Krebs ausgewählt ist aus Gehirnkrebs, Brustkrebs, Leberkrebs, Bauchspeicheldrüsenkrebs, Darmkrebs und Lungenkrebs.

4. Die mesenchymale Stammzelle zur Verwendung gemäß Anspruch 3, wobei die mesenchymale Stammzelle für die Verabreichung vor der Verabreichung von 5-Fluorocytosin ist.

## Revendications

1. Utilisation d'une cellule souche mésenchymateuse exprimant un gène suicide pour la fabrication d'un médicament de traitement d'un cancer,
le gène suicide étant un gène codant pour la cytosine désaminase,
la cytosine désaminase exprimée à partir du gène suicide convertissant la 5-fluorocytosine en 5-fluorouracile,
la cellule souche mésenchymateuse étant une cellule souche mésenchymateuse humaine isolée de la moelle épinière, du sang périphérique ou du sang du cordon, et
le cancer étant sélectionné parmi le cancer du cerveau, le cancer du sein, le cancer du foie, le cancer du pancréas, le cancer colorectal et le cancer du poumon.

2. Utilisation selon la revendication 1,
le médicament étant fabriqué pour l'administration avant l'administration de la 5-fluorocytosine.

3. Cellule souche mésenchymateuse exprimant un gène suicide pour l'utilisation dans le traitement d'un cancer chez un sujet,
le gène suicide étant un gène codant pour la cytosine désaminase,
la cytosine désaminase exprimée à partir du gène suicide convertissant la 5-fluorocytosine en 5-fluorouracile,
la cellule souche mésenchymateuse étant une cellule souche mésenchymateuse humaine isolée de la moelle épinière, du sang périphérique ou du sang du cordon, et
le cancer étant sélectionné parmi le cancer du cerveau, le cancer du sein, le cancer du foie, le cancer du pancréas, le cancer colorectal et le cancer du poumon.

4. Cellule souche mésenchymateuse pour l'utilisation selon la revendication 3,
la cellule souche mésenchymateuse étant destinée à une administration avant l'administration de la 5-fluorocytosine.


### SEQUENCE LISTING

<110> SUH, Hae-Young et al.
<120> USE OF MESENCHYMAL STEM CELLS GENETICALLY MODIFIED TO EXPRESS A SUICIDE GENE FOR TREATING A CANCER
<130> PCA60934/SHY
<150> KR 10-2005-0091155
   <151> 2005-09-29
<160> 4
<170> Kopatent In 1.71
<210> 1
   <211> 40
   <212> DNA
   <213> Artificial Sequence
<220>
   <223> Primer CD-F
<400> 1
   gaattcaggc tagcaatgtc tcgaataacg ctttacaaac 40
<210> 2
   <211> 26
   <212> DNA
   <213> Artificial Sequence
<220>
   <223> Primer CD-R
<400> 2
   ggattctcta gctggcagac agccgc 26
<210> 3
   <211> 24
   <212> DNA
   <213> Artificial Sequence
<220>
   <223> Primer for sequencing a CD gene
<400> 3
   catacgattt aggtgacact atag 24
<210> 4
   <211> 20
   <212> DNA
   <213> Artificial Sequence
<220>
   <223> Primer for sequencing a CD gene
<400> 4
   accgggaaac acctattgtg 20